# FASCICULE DE BREVET EUROPEEN

(11) **EP 2 171 459 B1**
(45) Date de publication et mention de la délivrance du brevet: **13.04.2016**
(21) Numéro de dépôt: 08831120.4
(22) Date de dépôt: 11.07.2008
(51) Int. Cl.: G01N 33/543, B03C 1/01, G01N 33/538

(54) **PROCEDE DE DOSAGE D'UN ANALYTE DANS UN MILIEU LIQUIDE**
VERFAHREN ZUR DOSIERUNG EINES ANALYTS IN EINEM FLÜSSIGMEDIUM
METHOD FOR DOSING AN ANALYTE IN A LIQUID MEDIUM

(30) Priorité: 27.07.2007 FR 0705530
(43) Date de publication de la demande: 07.04.2010
(73) Titulaire: Bertin Technologies, 78180 Montigny-le-Bretonneux (FR)
(72) Inventeur: COMPAIN, Eric, F- 13109 Simiane Collonge (FR); ROUZEAU, Catherine, F-78280 Guyancourt (FR); BIZET, Karine, F-78150 Le Chesnay (FR)
(74) Mandataire: Ramey, Daniel
(86) Numéro de dépôt international: PCT/FR2008/001023
(87) Numéro de publication internationale: WO 2009/034271

(56) Documents cités:
- WO-A-01/14591
- WO-A-02/35205
- WO-A-03/044532
- WO-A-03/054523
- WO-A-2005/010543
- WO-A-2005/026681
- US-A1- 2006 240 572

## Description

L'invention concerne un procédé de dosage d'au moins un analyte dans un milieu liquide, ce procédé comprenant une réaction de l'analyte avec des ligands greffés sur des particules magnétiques contenues dans le milieu liquide.

On connaît, par le document WO 03/044532, un procédé de dosage de ce type, qui consiste à appliquer un champ magnétique à un milieu liquide contenant l'analyte à doser et des particules magnétiques fonctionnalisées en surface avec des ligands spécifiques de l'analyte à doser. Le champ magnétique a une intensité suffisante pour provoquer la formation de chaînes ou d'amas de particules magnétiques et son application au milieu liquide est maintenue pendant une durée suffisante pour que l'analyte se couple ou s'associe avec les ligands des particules magnétiques, après quoi on annule le champ, ce qui provoque la séparation des particules magnétiques dont les ligands n'ont pas réagi avec l'analyte à doser. Par observation directe au microscope, ou de préférence par mesure d'un paramètre tel que la densité optique du milieu liquide, on peut déterminer la présence de l'analyte dans le milieu liquide. On peut aussi déterminer sa concentration par mesure de la variation de la densité optique du milieu liquide, entre un instant t0 correspondant au début de l'application du champ magnétique et un instant t1 qui suit l'annulation du champ magnétique à la fin de la réaction entre l'analyte et les ligands.

La valeur de la variation de la densité optique entre t0 et t1 est comparée à des courbes ou des valeurs d'étalonnage obtenues avec des concentrations connues de l'analyte à doser.

Ce procédé présente l'avantage d'avoir une limite de détection qui est plus faible que celle des procédés classiques d'agglutination exécutés en l'absence de champ magnétique.

Les documents WO 03/054523 A, US 2006/240572 A1 et WO 02/35205 A divulguent également des procédés de dosage d'un analyte dans un milieu liquide.

La présente invention a notamment pour objet des modifications de ce procédé qui permettent d'abaisser davantage sa limite de détection et d'améliorer sa robustesse.

Elle propose à cet effet un procédé de dosage d'un analyte dans un milieu liquide, consistant à placer dans ce milieu des particules magnétiques fonctionnalisées par des ligands spécifiques de l'analyte à doser, à faire agir sur le milieu liquide un champ magnétique permettant un accrochage des particules magnétiques et la formation d'amas de particules magnétiques, et à déterminer la présence et la concentration de l'analyte à partir de la variation d'un paramètre tel par exemple que la densité optique du milieu liquide, la valeur de ce paramètre étant mesurée avant l'application du champ magnétique, caractérisé en ce qu'il comprend une répétition de cycles comprenant une première phase d'application du champ magnétique au milieu liquide et une seconde phase où le champ magnétique est nul, le paramètre étant mesuré au début et à la fin de chaque cycle, la durée d'application du champ magnétique pendant chaque cycle étant inférieure à celle de la réaction entre l'analyte à doser et les ligands des particules magnétiques, le procédé consistant également à calculer à partir des mesures précitées une valeur limite de la variation du paramètre pour un temps infini d'application du champ magnétique et à en déduire la concentration de l'analyte dans le milieu liquide.

L'invention permet, de façon générale, d'améliorer le rapport signal/bruit des mesures et d'abaisser la limite de détection d'un facteur au moins égal à 5.

Le procédé selon l'invention comprend n répétitions du cycle d'application du champ magnétique et de mesure du paramètre pendant la durée de la réaction entre l'analyte et les ligands, n étant compris entre 2 et 100, et de préférence compris entre 5 et 60.

La durée totale d'application du champ magnétique pendant les n cycles est inférieure ou sensiblement égale à la durée de la réaction entre les ligands et l'analyte.

La durée d'application du champ magnétique pendant un cycle est comprise entre 1 seconde et 1 minute et est de préférence comprise entre 10 secondes et 50 secondes.

De préférence, la mesure de la valeur du paramètre précité est faite, à la fin de chaque cycle, durant un retour à l'équilibre dans le milieu liquide, c'est-à-dire après une dispersion au moins partielle des amas de particules magnétiques.

Le nombre de cycles n est déterminé de telle sorte que la durée totale d'application du champ magnétique soit égale à p fois la constante de temps de la variation du paramètre mesuré, cette variation étant du type exponentiel.

De préférence, p est égal à 2.

De façon plus générale, p est compris entre 0,5 et 5.

Selon une autre caractéristique de l'invention, le procédé consiste également à calculer la variation à la fin de chaque cycle, du paramètre mesuré pour une durée cumulée d'application du champ magnétique depuis le premier cycle réalisé, et à calculer par extrapolation une valeur limite vers laquelle tend la valeur calculée pour un temps infini d'application du champ magnétique.

Les calculs de variation du paramètre sont réalisés avantageusement en temps réel à la fin de chaque cycle.

En variante, lorsque cela est possible, ces calculs peuvent être réalisés après la fin de la réaction entre l'analyte et les ligands. Les calculs des variations du paramètre sur les durées cumulées depuis le premier cycle et de la variation du paramètre pour un temps infini d'application du champ magnétique sont couplés et on prend en compte les variations de constantes de temps qui sont dues à la réduction de mobilité du milieu liquide dans le calcul des variations du paramètre sur les durées cumulées d'application du champ magnétique.

Avantageusement, ce procédé consiste également à identifier et à filtrer des phénomènes parasites qui influent sur le paramètre mesuré. Le procédé consiste pour cela à décomposer mathématiquement l'évolution temporelle du paramètre mesuré dans une base de fonctions ayant des constantes de temps représentatives des cinétiques du système et à ne conserver que les fonctions ayant des constantes de temps caractéristiques de l'accroche spécifique des ligands et de l'analyte à doser. Ce traitement permet de filtrer les mesures du paramètre et d'améliorer la sélectivité du procédé.

L'invention sera mieux comprise et d'autres caractéristiques, détails et avantages de celle-ci apparaîtront plus clairement à la lecture de la description qui suit, faite à titre d'exemple en référence aux dessins annexés dans lesquels :

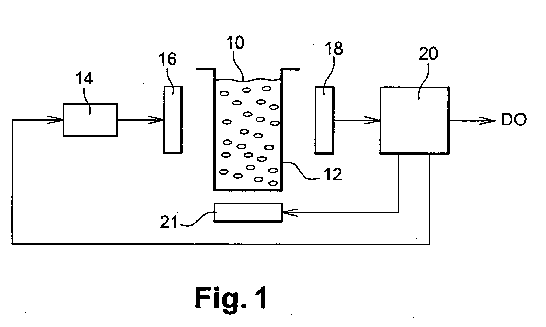

- la figure 1 représente schématiquement un dispositif de dosage selon l'invention ;
- la figure 2 est un graphe illustrant les principales étapes du procédé de dosage selon l'invention ;
- la figure 3 est un graphe illustrant les principales étapes d'une variante du procédé de dosage selon l'invention.

Comme déjà indiqué, ce procédé est, de façon générale, du même type que celui décrit dans le document WO 03/044532 et utilise des particules colloïdales (ayant des dimensions comprises de préférence entre 100 et 1000 nm) d'un matériau superparamagnétique, qui peuvent s'organiser rapidement en chaînes ou en amas de particules sous l'effet d'un champ magnétique et se séparer rapidement à la fin de l'application du champ magnétique.

Ces particules sont fonctionnalisées en surface avec des ligands spécifiques d'analytes à doser, la fonctionnalisation des particules magnétiques par des ligands étant réalisée de façon classique, comme décrit par exemple dans le document WO 03/044532.

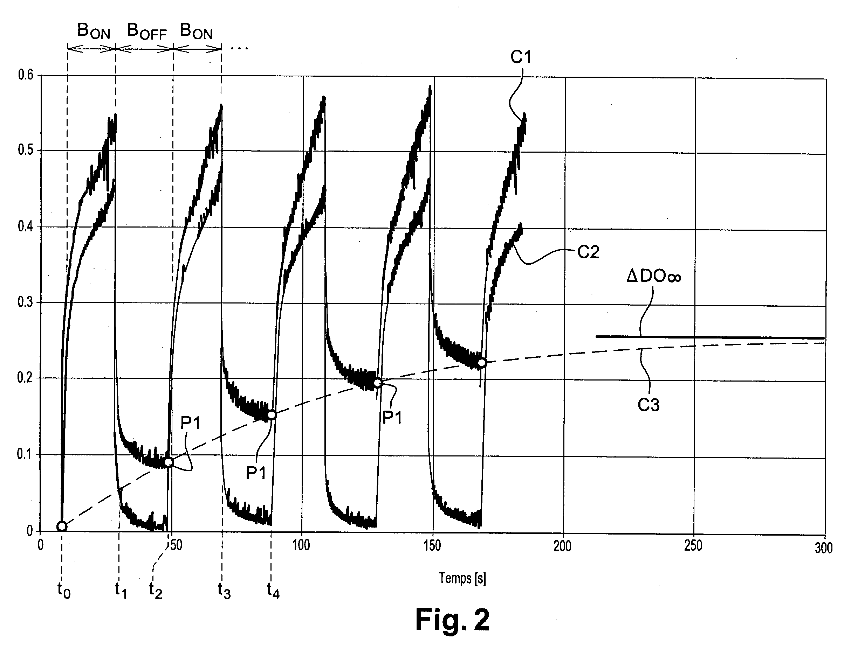

Un volume déterminé d'une solution 10 de particules colloïdales magnétiques fonctionnalisées est placé avec un échantillon d'analyte à doser dans un récipient approprié 12 en matériau plastique transparent. On fait agir un champ magnétique d'une intensité d'environ 10 à 40 mT sur le récipient 12 par exemple en le plaçant entre deux aimants permanents ou sur un électro-aimant 21 ou entre deux électro-aimants et on mesure la densité optique du milieu liquide 10 en utilisant pour cela une source lumineuse 14 telle par exemple qu'un générateur laser associé à une optique appropriée 16 pour éclairer le récipient 12 et un photodétecteur 18 tel par exemple qu'une photodiode, ou un ensemble de photodétecteurs, tel par exemple qu'une caméra CCD ou CMOS, dont la sortie est raccordée à une entrée de moyens 20 de traitement de l'information qui pilotent la source lumineuse 14 et les photodétecteurs 18 et qui fournissent en sortie un signal DO de densité optique du milieu liquide dont la variation en fonction du temps est représentée par le graphe de la figure 2. Les moyens 20 permettent également de contrôler des moyens d'application et de coupure du champ magnétique (par exemple l'électro-aimant 21), pour synchroniser les mesures avec la présence du champ magnétique au sein du milieu liquide 10.

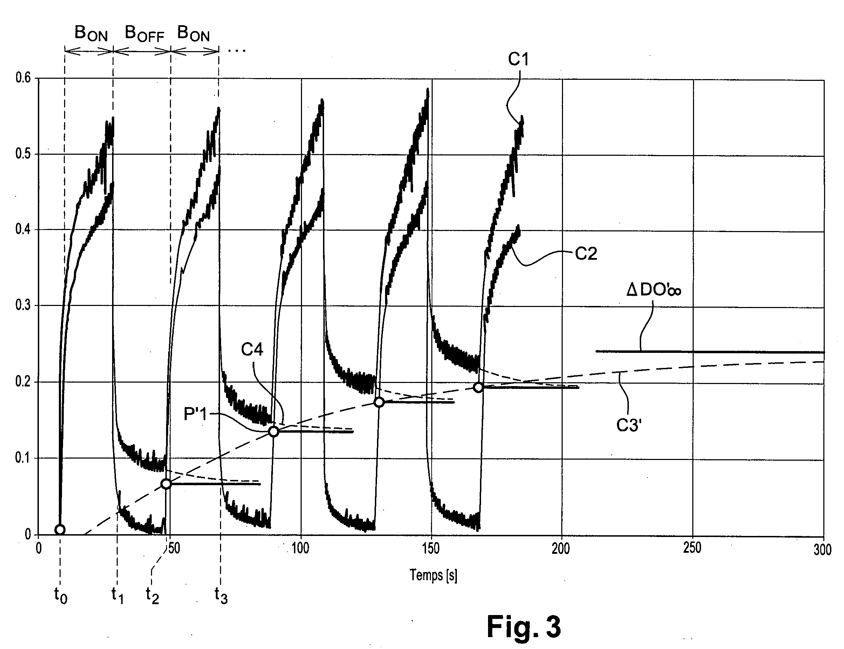

Selon l'invention, le champ magnétique est appliqué de façon cyclique au milieu liquide 10 contenant l'échantillon à doser, chaque cycle comprenant deux phases et ayant une durée qui est inférieure à la durée de réaction entre l'analyte et les ligands fixés sur les particules magnétiques. La première phase est caractérisée par l'application du champ magnétique (Bon en figure 2) et a une durée qui est supérieure au temps de formation des amas de particules magnétiques. La seconde phase est caractérisée par un champ magnétique nul (Boff en figure 2) et a une durée qui est au moins égale ou supérieure au temps de dispersion des amas de particules et si possible des accroches non spécifiques entre les particules et qui est inférieure à la constante de temps de dissociation des accroches spécifiques entre les ligands et l'analyte. La densité optique du milieu liquide est mesurée au début de chaque cycle et à la fin de chaque cycle. De préférence, la densité optique est échantillonnée pendant toute la durée du procédé, la période d'échantillonnage étant petite par rapport à la durée du cycle et aux constantes de temps caractéristiques du système. Elle est typiquement comprise entre 10 ms et 1 seconde. La durée de l'application du champ magnétique dans chaque cycle est déterminée pour que ces cycles puissent être répétés n fois pendant la durée de la réaction entre les ligands et l'analyte à doser. Ce paramètre n est de façon générale compris entre 2 et 100, de préférence entre 5 et 60, et il peut être en pratique égal à 20 ou 30 environ. Pour des raisons de simplification et de lisibilité des dessins, il est égal à 4 dans les figures 2 et 3.

La durée d'application du champ magnétique pendant chaque cycle est comprise de façon générale entre une seconde et une minute et de préférence entre 10 secondes et 50 secondes, en fonction de la durée totale de la réaction entre ligands et analytes.

Dans le graphe de la figure 2, la courbe C1 représente la variation de la densité optique du milieu liquide mesurée en nombre de décades d'atténuation de la lumière, en fonction du temps mesuré en secondes, dans le cas d'un milieu liquide contenant une forte concentration de l'analyte à doser, la courbe C2 représentant la variation de la densité optique du même milieu liquide contenant une très faible concentration d'analyte à doser.

Au temps t0, la densité optique du milieu liquide est mesurée une première fois en l'absence de champ magnétique, puis le champ magnétique est appliqué (phase Bon). La densité optique du milieu liquide augmente de façon très rapide puis le champ magnétique est coupé au temps t1, ce qui entraîne une diminution très rapide de la densité optique du milieu liquide (phase Boff). L'intervalle de temps t0-t1 est d'ici environ 20 secondes. La densité optique du milieu liquide est mesurée à nouveau au temps t2 après un retour partiel à l'équilibre dans le milieu liquide, l'intervalle t1-t2 étant d'environ 20 secondes, puis le champ magnétique est appliqué à nouveau pour une durée d'environ 20 secondes et est ensuite coupé à l'instant t3, la densité optique du milieu liquide étant à nouveau mesurée à l'instant t4 après un retour à l'équilibre, et ainsi de suite. Les cycles d'application et de coupure du champ magnétique et de mesure de la densité optique du milieu liquide sont répétés jusqu'à un avancement suffisant de la réaction entre les ligands et l'analyte à doser, la durée de cette réaction pouvant être de l'ordre de plusieurs minutes. Les acquisitions des valeurs du paramètre optique à chaque fin de cycle sont illustrées par les points P1. Ces points permettent de calculer par interpolation une courbe C3 de type exponentiel et d'extrapoler une valeur limite ΔDO∞ qui est celle que le paramètre atteindrait après un nombre infini de cycles.

Les valeurs P1 sont des valeurs de densité optique moyennées sur typiquement 5 secondes. Lorsque, comme dans le document précité WO 03/044532, la densité optique est mesurée une fois avant application du champ magnétique et une fois après application du champ, la durée totale d'acquisition de la mesure est d'environ 10 secondes. De plus, le procédé reste long car il faut attendre que le milieu liquide soit stabilisé complètement après coupure du champ magnétique.

Lorsque, conformément à l'invention, ces mesures sont répétées 30 fois pour l'acquisition des valeurs P1, le temps total d'acquisition est d'environ 300 secondes, ce qui permet de gagner un facteur égal à √30, soit d'environ 5 à 6, sur le rapport signal/bruit de la valeur ΔDO∞. Le bruit est ici, entre autres, le bruit optique, qui est dû à l'agitation des particules dans le milieu liquide, qui rentrent et qui sortent du volume de mesure et le bruit de la chaîne d'acquisition de la mesure comprenant la source lumineuse 14, les photodétecteurs 18 et les moyens de numérisation et de traitement du signal. En outre, l'extrapolation de la valeur limite permet de réduire la durée du procédé tout en limitant l'influence des variations des constantes de temps dues par exemple à la température.

Cette amélioration du rapport signal/bruit permet d'abaisser de façon significative la limite de détection du procédé de dosage.

On peut encore abaisser cette limite de détection en utilisant la variante du procédé illustrée par la figure 3. Cette variante tient compte des phénomènes de dérive lente tels que la sédimentation, la dérive thermique des moyens de mesure et des accroches non spécifiques, c'est-à-dire des particules magnétiques colloïdales qui se sont accrochées de façon temporaire sans qu'il y ait de réaction entre les ligands qu'elles comportent et l'analyte à doser.

Pour cela, à la fin de chaque cycle d'application du champ magnétique et de mesure de la densité optique, on calcule par extrapolation (au moyen d'une courbe C4) une valeur limite P'1 de la décroissance de la densité optique du milieu liquide suite à une application de champ cumulée depuis le début du premier cycle et après un retour à l'équilibre du milieu liquide. L'extrapolation permet d'optimiser le rapport signal/bruit en utilisant plus de mesures et de réduire les temps de cycle.

A l'issue des n cycles d'application du champ magnétique et de mesure de la densité optique, on calcule par extrapolation des valeurs P'1 et par filtrage des phénomènes parasites une courbe C'3 passant par les points P'1 et une valeur limite ΔDO'∞ de la variation de la densité optique due aux accroches spécifiques, c'est-à-dire aux réactions entre les ligands des particules magnétiques et l'analyte à doser.

Le calcul prend en compte les n valeurs P'1 calculées précédemment de la variation de la densité optique mesurée, pour des durées cumulées d'application du champ magnétique, et l'extrapolation est faite pour une durée infinie d'application du champ magnétique. Le filtrage consiste à ne garder dans les termes de croissance de la variation de densité optique que ceux dont les constantes de temps correspondent aux accroches spécifiques. Cela permet d'éliminer un certain nombre de phénomènes parasites qui ont été identifiés lors des opérations d'étalonnage et dont les constantes de temps ne correspondent pas à la réaction ligands/analyte à doser. En particulier, les accroches non spécifiques correspondent à des forces de liaison plus faibles et ont des constantes de dissociation plus rapide. Les constantes de temps de la réaction ligands / analyte à doser sont étalonnées au préalable pour optimiser la sélectivité.

Le nombre de cycles d'application du champ magnétique et de mesure de la densité optique est choisi de préférence pour que la durée totale d'application du champ magnétique soit égale à quelques fois la constante de temps de la courbe de variation de la densité optique due aux accroches spécifiques, la durée totale d'application du champ magnétique étant idéalement égale à deux fois cette constante de temps.

On peut ainsi réaliser la plupart des calculs en temps réel pendant la durée de la réaction entre les ligands et l'analyte à doser et on limite le post-traitement au calcul de la valeur limite ΔDO'∞ de la variation de la densité optique due aux accroches spécifiques.

En variante, et si la capacité de calcul des moyens de traitement 20 et la durée totale de la mesure le permettent, on peut réaliser les calculs entièrement à la fin de la réaction de dosage. Dans ce cas, les algorithmes de calcul des valeurs limites P'1 de la variation de la densité optique mesurée à la fin de chaque cycle et de la variation de la densité optique ΔDO'∞ due aux accroches spécifiques sont couplés de façon à pouvoir prendre en compte dans le calcul des valeurs P'1 les évolutions de constantes de temps qui sont dues à la réduction de mobilité du milieu liquide, ce phénomène étant mesurable à partir de la variation de la densité optique due aux accroches spécifiques.

De façon générale, l'invention est applicable à la détection d'antigènes de tout type à l'aide de ligands spécifiques naturels ou synthétiques de tout type. Elle permet d'abaisser la limite de détection d'un analyte d'un facteur au moins égal à cinq.

Par exemple, dans le cas de la détection d'un fragment recombinant de la toxine botulique à l'aide d'anticorps monoclonaux, l'invention a permis d'abaisser la limite de détection de 2.10⁻¹¹ à 4.10⁻¹² mole d'analyte par litre de milieu liquide.

## Revendications

1. Procédé de dosage d'un analyte dans un milieu liquide, consistant à placer dans ce milieu des particules magnétiques fonctionnalisées par des ligands spécifiques de l'analyte à doser, à faire agir sur le milieu liquide un champ magnétique permettant un accrochage des particules magnétiques et la formation d'amas de particules magnétiques, et à déterminer la présence et la concentration de l'analyte à partir de la variation d'un paramètre tel par exemple que la densité optique du milieu liquide, la valeur de ce paramètre étant mesurée avant l'application du champ magnétique, **caractérisé en ce qu'**il comprend une répétition de cycles comprenant une première phase d'application du champ magnétique au milieu liquide et une seconde phase où le champ magnétique est nul, le paramètre étant mesuré au début et à la fin de chaque cycle , la durée d'application du champ magnétique pendant chaque cycle étant inférieure à celle de la réaction entre l'analyte à doser et les ligands des particules magnétiques, le procédé consistant également à calculer à partir des mesures précitées une valeur limite de la variation du paramètre pour un temps infini d'application du champ magnétique et à en déduire la concentration de l'analyte dans le milieu liquide.

2. Procédé selon la revendication 1, **caractérisé en ce qu'**il comprend n cycles d'application du champ magnétique et de mesure du paramètre pendant la durée de la réaction entre l'analyte et les ligands, n étant compris entre 2 et 100.

3. Procédé selon la revendication 2, **caractérisé en ce que** n est compris entre 5 et 60.

4. Procédé selon l'une des revendications précédentes, **caractérisé en ce que** la durée d'application du champ magnétique pendant un cycle est comprise entre 1 seconde et 1 minute.

5. Procédé selon la revendication 4, **caractérisé en ce que** la durée d'application du champ magnétique pendant un cycle est comprise entre 10 secondes et 50 secondes.

6. Procédé selon l'une des revendications 1 à 5, caractérisé en qu'il consiste à mesurer la valeur du paramètre précité à la fin de chaque cycle, après un retour à l'équilibre dans le milieu liquide.

7. Procédé selon l'une des revendications précédentes, **caractérisé en ce qu'**il consiste à déterminer le nombre de cycles n de telle sorte que la durée totale d'application du champ magnétique soit égale à p fois la constante de temps de la variation du paramètre mesuré, cette variation étant du type exponentiel.

8. Procédé selon la revendication 7, **caractérisé en ce que** p est égal à 2.

9. Procédé selon l'une des revendications précédentes, **caractérisé en ce qu'**il consiste à calculer la variation, à la fin de chaque cycle, du paramètre mesuré pour une durée cumulée d'application du champ magnétique depuis le premier cycle réalisé, et à calculer par extrapolation une valeur limite vers laquelle tend la valeur calculée pour un temps infini d'application du champ magnétique.

10. Procédé selon l'une des revendications précédentes, **caractérisé en ce que** les calculs de variation du paramètre sont réalisés en temps réel à la fin de chaque cycle.

11. Procédé selon la revendication 9, **caractérisé en ce que** les calculs de variation du paramètre sont réalisés après la fin de la réaction entre l'analyte et les ligands, **en ce que** les calculs des variations du paramètre sur les durées cumulées depuis le premier cycle et de la variation du paramètre pour un temps infini d'application du champ magnétique sont couplés et **en ce qu'**on prend en compte les variations de constantes de temps dues à la réduction de mobilité du milieu liquide dans le calcul des variations du paramètre sur les durées cumulées d'application du champ magnétique.

12. Procédé selon l'une des revendications précédentes, **caractérisé en ce qu'**il consiste à identifier et à éliminer par filtrage des phénomènes parasites influant sur le paramètre mesuré, et pour cela à mesurer les constantes de temps de la réaction des ligands et de l'analyte pour des concentrations élevées d'analyte, à décomposer l'évolution temporelle des valeurs mesurées dans une base de fonctions ayant des constantes de temps différentes et à ne conserver que les composantes des valeurs mesurées ayant des constantes de temps correspondant à celles de la réaction ligands-analyte.

## Patentansprüche

1. Verfahren zur Dosierung eines Analyten in einem flüssigen Medium, welches darin besteht, dass man in dieses Medium magnetische Partikel platziert, die mit spezifischen Liganden des zu dosierenden Analyten funktionalisiert sind, und darin, auf das flüssige Medium ein Magnetfeld einwirken zu lassen, welches das Anhaften der Magnetpartikel und die Bildung von magnetischen Partikelclustern ermöglicht, und darin, die Präsenz und die Konzentration des Analyten ausgehend von der Änderung eines Parameters zu bestimmen, wie beispielsweise die optische Dichte des flüssigen Mediums, wobei der Wert dieses Parameters vor der Anwendung des Magnetfelds gemessen wird, **dadurch gekennzeichnet, dass** es eine Wiederholung von Zyklen bestehend aus einer ersten Anwendungsphase des Magnetfels im flüssigen Medium und einer zweiten Phase, in der das Magnetfeld gleich Null ist, aufweist, wobei der Parameter zu Beginn und am Ende jedes Zyklus gemessen wird, und die Anwendungsdauer des Magnetfelds während eines jeden Zyklus kürzer ist als die der Reaktion zwischen dem zu dosierenden Analyten mit den Liganden der magnetischen Partikel, wobei das Verfahren ferner darin besteht, ausgehend von den vorgenannten Messungen einen Grenzwert für die Änderung des Parameters bei einer zeitlich unbegrenzten Anwendung des Magnetfelds zu berechnen und daraus die Konzentration des Analyten im flüssigen Medium abzuleiten.

2. Verfahren nach Anspruch 1, **dadurch gekennzeichnet, dass** es während der Dauer der Reaktion zwischen dem Analyten und den Liganden n Zyklen bestehend aus Magnetfeldanwendung und Parametermessung umfasst, wobei n zwischen 2 und 100 liegt.

3. Verfahren nach Anspruch 2, **dadurch gekennzeichnet, dass** n zwischen 5 und 60 liegt.

4. Verfahren nach einem der vorhergehenden Ansprüche, **dadurch gekennzeichnet, dass** die Anwendung des Magnetfelds während eines Zyklus zwischen 1 Sekunde und 1 Minute dauert.

5. Verfahren nach Anspruch 4, **dadurch gekennzeichnet, dass** die Anwendung des Magnetfelds während eines Zyklus zwischen 10 Sekunden und 50 Sekunden dauert.

6. Verfahren nach einem der Ansprüche 1 bis 5, dadurch gekenntzeichnet, dass der Wert des vorgenannten Parameters am Ende eines jedes Zyklus, nachdem das flüssige Medium wieder sein Gleichgewicht gefunden hat, gemessen wird.

7. Verfahren nach einem der vorhergehenden Ansprüche, **dadurch gekennzeichnet, dass** es darin besteht, die Anzahl der Zyklen n so festzulegen, dass die Gesamtanwendungsdauer des Magnetfelds gleich P mal die Zeitkonstante der Änderung des gemessenen Parameters ist, wobei diese Änderung exponentiell verläuft.

8. Verfahren nach Anspruch 7, **dadurch gekennzeichnet, dass** p gleich 2 ist.

9. Verfahren nach einem der vorhergehenden Ansprüche, **dadurch gekennzeichnet, dass** es darin besteht, am Ende eines jeden Zyklus die Änderung des gemessenen Parameters für eine kumulierte Anwendungsdauer des Magnetfelds vom ersten Zyklus ab zu berechnen, und darin, mittels Extrapolation einen Grenzwert zu berechnen, dem sich der für eine unbestimmte Anwendungsdauer des Magnetfels berechnete Wert nähert.

10. Verfahren nach einem der vorhergehenden Ansprüche, **dadurch gekennzeichnet, dass** die Änderungen des Parameters am Ende eines jeden Zyklus in Echtzeit berechnet werden.

11. Verfahren nach Anspruch 9, **dadurch gekennzeichnet, dass** die Änderungen des Parameters nach Beendigung der Reaktion zwischen Analyt und Ligand berechnet werden, dass die Berechnungen der Parameteränderungen einer kumulierten Dauer ab dem ersten Zyklus und der Parameteränderungen einer unbestimmten Anwendungsdauer des Magnetfelds aneinander gekoppelt sind, und dass die durch die Verringerung der Beweglichkeit des flüssigen Mediums herbeigeführten Änderungen der Zeitkonstanten bei der Berechnung der Parameteränderungen der kumulierten Anwendungsdauer des Magnetfels berücksichtigt werden.

12. Verfahren nach einem der vorhergehenden Ansprüche, **dadurch gekennzeichnet, dass** die auf den gemessenen Parameter einwirkenden Störeinflüsse durch Filterung identifiziert und beseitig und hierfür die Konstanten der Reaktionsdauer zwischen Liganden und Analyt bei erhöhten Konzentrationen des Analyten gemessen werden, die zeitliche Entwicklung der Messwerte in eine Funktionsbasis mit unterschiedlichen Zeitkonstanten zerlegt und nur die Komponenten jener Messwerte beibehalten werden, deren zeitliche Konstanten mit jenen der Ligand-Analyt-Reaktion übereinstimmen.

## Claims

1. Method of quantifying an analyte in a liquid medium, which consists in placing magnetic particles in this medium, which have been functionalized by specific ligands of the analyte being quantified, in causing a magnetic field to act on the liquid medium, thereby enabling adhesion of the magnetic particles and the formation of clusters of magnetic particles, and in determining the presence and concentration of the analyte from the variation of a parameter such as the optical density of the liquid medium, for example, the value of this parameter being measured prior to application of the magnetic field, **characterised in that** it includes repeated cycles comprising a first phase of applying the magnetic field to the liquid medium and a second phase where the magnetic field is zero, the parameter being measured at the beginning and at the end of each cycle, the magnetic field application period during each cycle being shorter than that of the reaction between the analyte being quantified and the ligands of the magnetic particles, the method likewise consisting in calculating, on the basis of the aforesaid measurements, a limiting value for the variation in the parameter for an infinite magnetic field application time, and in deducing therefrom the concentration of the analyte in the liquid medium.

2. Method of claim 1, **characterised in that** it includes n cycles of applying the magnetic field and of measuring the parameter over the duration of the reaction between the analyte and the ligands, n being between 2 and 100.

3. Method of claim 2, **characterised in that** n is between 5 and 60.

4. Method as claimed in one of the preceding claims, **characterised in that** the magnetic field application time during one cycle is between 1 second and 1 minute.

5. Method of claim 4, **characterised in that** the magnetic field application time during one cycle is between 10 seconds and 50 seconds.

6. Method as claimed in one of claims 1 to 5, **characterised in that** it consists in measuring the aforesaid parameter at the end of each cycle, after a return to equilibrium in the liquid medium.

7. Method as claimed in one of the preceding claims, **characterised in that** it consists in determining the number of cycles n such that the total magnetic field application time is equal to p times the time constant of the variation in the parameter measured, this variation being of the exponential type.

8. Method of claim 7, **characterised in that** p is equal to 2.

9. Method as claimed in one of the preceding claims, **characterised in that** it consists in calculating the variation in the measured parameter, at the end of each cycle, for a magnetic field application time accumulated from the first cycle carried out, and in calculating, by extrapolation, a limiting value that the calculated value approaches for an infinite magnetic field application time.

10. Method as claimed in one of the preceding claims, **characterised in that** the calculations of the variation in the parameter are carried out in real time, at the end of each cycle.

11. Method of claim 9, **characterised in that** the calculations of the variation in the parameter are carried out at the end of the reaction between the analyte and the ligands, **in that** the calculations of the variations in the parameter over the time periods accumulated from the first cycle and of the variation in the parameter for an infinite magnetic field application time are paired, and **in that**, in calculating the variations in the of the parameter over the cumulative magnetic field application times, account is take of the reduction in the mobility of the liquid medium.

12. Method as claimed in one of the preceding claims, **characterised in that** it consists in identifying and in eliminating by filtration the parasitic phenomena influencing the measured parameter, and, in order to accomplish this, in measuring the time constants for the reaction of the ligands and the analyte, for high analyte concentrations, in breaking down the temporal evolution of the measured values in a functions base having different time constants and in retaining only those components of the measured values which have time constants corresponding to those of the ligands-analyte reaction.
